# EUROPÄISCHE PATENTSCHRIFT

(11) **EP 3 908 133 B1**
(45) Veröffentlichungstag und Bekanntmachung des Hinweises auf die Patenterteilung: **11.10.2023**
(21) Anmeldenummer: 19836461.4
(22) Anmeldetag: 10.12.2019
(51) Int. Cl.: A61K 31/05, A61K 36/185, A61M 11/00, A61M 11/02, A61M 11/04, A61M 11/06, A61M 16/00, G01N 33/94, A24F 40/42, A61M 15/00, A61J 3/00, A61M 15/06, A61K 31/352

(54) **VORRICHTUNG ZUM ERZEUGEN EINES GASFÖRMIGEN WIRKSTOFFS ODER EINES GASFÖRMIGEN WIRKSTOFFGEMISCHS**
DEVICE FOR THE EXTRACTION AND ASPIRATION OF ACTIVE SUBSTANCES, IN PARTICULAR FROM THE CANNABIS PLANT
DISPOSITIF POUR L'EXTRACTION ET L'ASPIRATION DE SUBSTANCES ACTIVES, EN PARTICULIER DE CANNABIS

(30) Priorität: 07.01.2019 DE 102019000016; 07.01.2019 DE 102019000018; 15.01.2019 DE 102019000199
(43) Veröffentlichungstag der Anmeldung: 17.11.2021
(73) Patentinhaber: LuxCan Innovation S.A., 1273 Luxembourg-Hamm (LU)
(72) Erfinder: SCHMITT, Fritz, 1855 Luxemburg (LU)
(74) Vertreter: Angerhausen, Christoph
(86) Internationale Anmeldenummer: PCT/DE2019/101065
(87) Internationale Veröffentlichungsnummer: WO 2020/143863

(56) Entgegenhaltungen:
- WO-A1-2016/033242
- WO-A1-2018/216019
- WO-A1-2019/003118
- GB-A- 2 561 266
- JP-A- 2008 104 966
- US-A- 5 865 186
- US-A1- 2004 118 396
- US-A1- 2012 103 326
- US-A1- 2014 144 429
- US-A1- 2014 182 587
- US-A1- 2017 196 262
- Mirko Heinemann: "Wirtschaftsfaktor Cannabis? - Legalisierung könnte einen Wirtschaftsboom auslösen (Archiv)", , 18. August 2015 (2015-08-18), XP055678428, Gefunden im Internet: URL:https://www.deutschlandfunkkultur.de/w irtschaftsfaktor-cannabis-legalisierung-ko ennte-einen.976.de.html?dram:article_id=32 8530 [gefunden am 2020-03-20]

## Beschreibung

Die vorliegende Erfindung betrifft ein Kit zum Freisetzen und der Aufnahme eines pharmakologisch aktiven Wirkstoffs in der Lunge sowie ein Verfahren zum Erzeugen eines gasförmigen WirkstoffeWirkstoffs oder eines gasförmigen Wirkstoffgemischs, umfassend dieses Kit.

### HINTERGRUND DER ERFINDUNG

Die Legalisierung von Cannabis und die Verwendung als Medikament werfen viele Fragen zur sicheren und einfachen Applikation und zum Bereitstellen geeigneter Applikationsformen durch Apotheker und Ärzte auf.

Aus dem Stand der Technik sind eine Vielzahl unterschiedlicher Geräte bekannt, die versuchen, mit Heißluft, Heizplatten oder Glühdrähten Stoffe, Kräuter oder Flüssigkeiten bei einer spezifischen Temperatur so zu erhitzen, dass der gewünschte Wirkstoffe zur Inhalation zur Verfügung steht. Die meisten bekannten Vorrichtungen funktionieren nach dem "Herdplatten-Prinzip". Das heißt, dass Pflanzenteile, die einen Wirkstoff enthalten, auf einer erhitzten Fläche verteilt werden, in der Hoffnung, dass dabei eine Verdampfung der Inhaltsstoffe erfolgt. Diese Methode funktioniert nur unzureichend, da die in unmittelbarem Kontakt mit der erhitzten Fläche stehenden Substanzen wesentlich stärker erhitzt werden als die Substanzen in den darüberlegenden Schichten, wodurch eine gleichmäßige Verdampfung der Inhaltsstoffe nicht möglich ist.

Bei einem anderen Funktionsprinzip werden Pflanzenteile mit auf einen bestimmten Temperaturbereich erhitzter Luft durchströmt. Die Pflanzenteile dampfen daraufhin - die notwendige Mindesttemperatur vorausgesetzt - ihre Inhaltsstoffe aus, welche dann nach Abkühlung inhaliert werden können. Insbesondere bei einer Verwendung im medizinischen Bereich können diese Geräte keine Gerätesicherheit garantiert. Es fehlen alle Voraussetzungen für ein medizinisches Gerät, da das Gerät nicht sterilisiert werden kann. Dies ist allerdings dringend notwendig berücksichtigt man die zu erwartenden Verbrennungsrückstände auf den Heizplatten ("Grillrosten").

Die WO 2016/033242 A1 beschreibt ein Kit, umfassend a) einen Beutel mit einem Auslass, wobei der Auslass verschließbar und öffenbar ist; b) einen festen und/oder flüssigen Wirkstoffvorläufer; und c) Mittel zum Freisetzen eines gasförmigen Wirkstoffs oder eines gasförmigen Wirkstoffgemischs aus dem Wirkstoffvorläufer.

Es ist daher die Aufgabe der vorliegenden Erfindung ein Verfahren zur Freisetzung von Wirkstoffen aus entsprechenden Vorläufergemischen bereitzustellen, das Nachteile des Stands der Technik überwindet, insbesondere geeignet ist, Wirkstoffe in schonender Weise aus entsprechenden Vorläufergemischen freizusetzen, mit Mitteln, die medizinischen Standards genügen.

### BESCHREIBUNG DER ERFINDUNG

Diese Aufgabe wird gelöst durch die Merkmale der unabhängigen Ansprüche 1 und 6. Das erfindungsgemäße Kit ermöglicht es in einfacher und schonender Weise, aus einem festen und/oder flüssigen Wirkstoffvorläufer einen gasförmigen Wirkstoff freizusetzen. Hierbei kann vorgesehen sein, dass der Wirkstoff ein pharmakologisch aktiver Wirkstoff ist. Nach dem Freisetzen kann der gasförmige Wirkstoff, bzw. das gasförmige Wirkstoffgemisch, für medizinische Zwecke aspiriert oder eingeatmet werden. Es ist bekannt, dass das Einatmen eines Wirkstoffs besonders effizient ist, da hierdurch der Weg durch den Magen (bei oraler Gabe) oder den Pfortaderkreislauf (bei Injektion) gespart wird. Darüber hinaus wird die Wirkstoffaufnahme durch eine Aufnahme über die Mundschleimhaut unterstützt. Das erfindungsgemäße Kit und das erfindungsgemäße Verfahren (wie weiter unten beschrieben) ermöglichen es dem Patienten, einen pharmakologisch aktiven Wirkstoff in der Lunge aufzunehmen, der dort lokal begrenzt wirkt oder rasch über die Alveolen in den Blutkreislauf gelangen und dort systematisch wirkt. Bei der Lokaltherapie können Nebenwirkungen im restlichen Organismus verringert werden und es wird eine geringere Dosis benötigt, da ein größerer Dosisanteil den Wirkungsort erreicht als bei peroraler Applikation. Aber auch wenn der Wirkstoff zur systemischen Wirkung in den Blutkreislauf gelangen soll, ist meist eine geringere Dosis notwendig, da der First-Pass-Effekt in der Leber umgangen wird. Aufgrund der großen Resorptionsfläche der Lunge (ca. 70-100 Quadratmeter) und der dünnen Epithelschicht gelangen Wirkstoffe schneller in den Körper und können früher wirken als bei peroraler Verabreichung.

Ein weiterer Vorteil des erfindungsgemäßen Kits liegt in seiner Flexibilität Der Wirkstoffvorläufer kann, abhängig vom Patienten und seinen Bedürfnissen, individuell und auf den Patienten abgestimmt durch den Apotheker zubereitet und dann in dem Kit zur Applikation bereitgestellt werden.

In einer Ausführungsform kann vorgesehen sein, dass der Wirkstoffvorläufer in dem Beutel angeordnet ist. Die Menge des Wirkstoffvorläufers kann patientenabhängig derart angepasst werden, dass die Menge an gasförmigen Wirkstoff, die aus dem Wirkstoffvorläufer freigesetzt wird, etwa dem Lungenvolumen des Patienten (beispielsweise Lungenvolumen +/- 10 %Vol.) entspricht. In diesem Zusammenhang kann vorgesehen sein, dass die Menge an gasförmigem Wirkstoff, der freigesetzt wird, das 10-fache, vorzugsweise das 20-fache, besonders bevorzugt das 30-fache des Gesamtvolumens des Beutels ist. Hierdurch kann eine auf den Patienten abgestimmte besonders effektive Applikation des gasförmigen Wirkstoffs erreicht werden.

In einer Ausführungsform kann vorgesehen sein, dass der Wirkstoffvorläufer eine Cannabisblüte, Marihuana, Haschisch, Haschischöl, zumindest ein Cannabinoid oder eine Mischung davon umfasst.

Cannabis (Hanf) gehört zusammen mit der Gattung Humulus (Hopfen) der Familie der Cannabisaceae an, wobei jedoch Humulus keine Cannabinoide enthält Innerhalb der Gattung Cannabis erfolgt eine botanische und chemotaxonomische Differenzierung und zwar in den Arten Cannabis sativa Linnaeus, Cannabis indica LAM und Cannabis ruderalis oder in die "Sammelart" Cannabis sativa L, bestehend aus den Unterarten Cannabis sativa ssp. sativa und ssp. indica. Darüber hinaus wird Cannabis in einen Drogen- und Faserhanf unterschieden, wobei die Unterscheidung aufgrund des mengenmäßigen Verhältnisses der Hauptcannabinoide Cannabidiol (CBD) und Δ⁹-Tetrahydrocannabinol (Δ⁹-THC) erfolgt (INN: Dronabinol). Faserhanf (auch: Nutzhanf, industrial hemp) wird hauptsächlich zur industriellen Fasergewinnung herangezogen und darf maximal ein Δ⁹-THC-Gehalt von 0,2 % aufweisen (z.B. Deutschland u.a.), während der Drogentyp ein Δ⁹-THC-Gehalt von ca. 5-15 % aufweisen kann (Marihuana, Haschisch). Cannabis sativa L, enthält über 400 verschiedene Inhaltsstoffe, davon gehören mehr als 60 Verbindungen der Klasse der Cannabinoide an. Die wichtigsten Cannabinoide sind im Folgenden dargestellt:
o *Carmabigerol-artige (CBG)* : Cannabigerol ((E)-CBG-C₅), Cannabigerol Monomethylether ((E)-CBGM-C₅ A), Cannabinerolsäure A ((Z)-CBGA-C₅ A), Cannabigerovarin ((E)-CBGV-C₃), Cannabigerolsäure A ((E)-CBGA-C₅ A), Cannabigerolsäure A Monomethylether ((E)-CBGAM-C₅ A), Cannabigerovarinsäure A ((E)-CHGVA-C₃ A) ;
o Cannabichromen-artige *(CBC)* : Cannabichromen (CBC-C₅), Cannabichromensäure A (CBCA-C₅ A), Cannabichromevarin (CBCV-C₃), Cannabichromevarinsäure A (CBCVA-C₃ A);
o *Cannabidiol-artige (CBD):* Cannabidiol (CBD-C₅), Cannabidiol Monomethylether (CBDM-C₅), Cannabidiol-C₄ (CBD-C₄), Cannabidivarin (CBDV-C₃), Cannabidiorcol (CBD-C₁), Cannabidiolsäure (CBDA-C₅), Cannabidivarinsäure (CBDVA-C₃);
o *Carmabinodiol-artige (CBND)* : Cannabinodiol (CBND-C₅), Cannabinodivarin (CBND-C₃);
o *Tetrahydrocannabinol*-*artige (THC)*: Δ₉-Tetrahydrocannabinol (Δ₉-THC-C₅), Δ₉-Tetrahydrocannabinal-C₄ (Δ₉-THC-C₄), Δ₉-Tetrahydrocannabivarin (Δ₉-THCV-C₃), Δ₉-Tetrahydrocannabiorool (Δ₉-THCO-C₁), Δ₉-Tetrahydrocannabinolsäure (Δ₉-THCA-C₅ A), Δ₉-Tetrahydrocannabinolsäure B (Δ₉-THCA-C₅ B), Δ₉-Tetrahydracannabinolsäure-C₄ (Δ₉-THCA-C₄ A und/oder B), Δ₉-Tetrahydrocannabivarinsäure A (Δ₉-THCVA-C₃ A), Δ₉-Tetrahydrocannabiorcolsäure (Δ₉-THCOA-C₁ A und/oder B), (-)-Δ8-trans-(6aR,10aR)-Δ8-Tetrahydrocannabinol (Δ8-THC-C₅),(-)-Δ8-trans-(6aR,10aR)-Tetrahydrocannabinoksäure A (Δ8-THCA-C₅ A); (-)-(6aS,10aR)-Δ₉-Tetrahydrocannabinol ((-)-cis-Δ₉-THC-C₅);
o *Cannabinol-artige (CBN):* Cannabinol CBN-C₅, Cannabinol-C₄ (CBN-C₄), Cannabivarin (CBN-C₃), Cannabinol-C₂ (CBN-C₂), Cannabiorool (CBN-C₁), Cannabinolsäure A (CBNA-C₅ A), Cannabinolmethylether (CBNM-C₅)
o *Cannabitriol-artige (CBT)* : (-)-(9R,10R)-trans-Cannabitriol ((-)-trans-CBT-C₅), (+)-(9S,10S)-Cannabitriol ((+)-trans-CBT-C₅), (±)-(9R,10S/9S,10R)-Cannabitriol ((±)-cis-CBT-C₅), (-)-(9R,10R)-trans[10-o-Ethyl-cannabitriol] ((-)-trans-CBT-OEt-C₅), (±)-(9R,10R/9S,10S)-Cannabitriol-C₃ ((±)-trans-CBT-(C₃),8,9-Dihydroxy-Δ6a(10a) tetrahydrocannabinol (8,9-Di-OH-CBT-C₅), Cannabidiolsäure A (CBDA-C₅ 9-OH-CBT-C5 ester), (-)-(6aR,9S,10S,10aR)-9,10-Dihydroxy-hexahydrocannabinol, Cannabiripsol Cannabiripsol-Cs, (-)-6a,7,10a-Trihydroxy-Δ₉-tetrahydrocannabinol ((-)-Cannabitetrol), 10-Oxo-Δ6a(10a) tetrahydrocannabinol (OTHC);
o *Cannabielsoin-artige (CBE)* : (5aS, 6S, 9R, 9aR) - C₅-Cannabielsoin (CBE-C₅), (5aS,6S,9R,9aR)-C₃₋Cannabielsoin (CBE-C₃), (5aS,6S,9R,9aR)-Cannabielsoinsäure A (CBEA-C₅ A), (5aS,6S,9R,9aR)-Cannabielsoinsäure B (CBEA-C₅ B), (5aS,6S,9R,9aR)-C3-Cannabielsoinsäure B (CBEA-C₃ B), Cannabiglendol-C₃ (OH-iso-HHCV-C₃), Dehydrocannabifuran (DCBF-C₅), Cannabifuran (CBF-C₅) ;
o *Isocannabinoide:* (-)-Δ7-trans-(1R,3R,6R)-Isotetrahydrocannabinol, (±)-Δ7-1,2-cis-(1R,3R,6S/1S,3S,6R)-Isotetrahydrocannabivarin, (-)-Δ7-trans-(1R,3R,6R)-Isotetrahydrocannabivarin;
o *Cannαbicyclol-artige (CBL)* : (±)-(1aS,3aR,8bR,8cR-Cannabicyclol (CBL-C₅), (±)-(1aS,3aR,8bR,8cR-Cannabicyclolsäure A (CBLA-C₅ A), (±)-(1aS,3aR,8bR,8cR-Cannabicyclovarin (CBLV-C₃);
o *Cannabicitran-artige (CBT)* : Cannabicitran (CBT-C₅) ;
o *Cannabichromanon-artige (CBCN)* : Cannabichromanon (CBCN-C₅), Cannabichromanon-C₃ (CBCN-C₃), Cannabicoumaronon (CBCON-C₅).

Neben den o. g. erwähnten Cannabinoiden finden sich deren zugehörige Carbonsäuren in der Rohdroge. Diese Carbonsäuren sind biosynthetische Precursors.

Cannabiszubereitungen üben eine Vielzahl therapeutischer Wirkungen aus, darunter antispastische, analgetische, antiemetische, neuroprotektive, antiinflammatorische sowie Wirkungen bei psychiatrischen Erkrankungen (Grotenhermen F, Müller-Vahl K: The therapeutic potential of cannabis and cannabinoids.

In Deutschland ist seit 2011 ein Cannabisextrakt, der THC (Dronabinol) und CBD im Verhältnis 1:1 enthält (Nabiximols), für die Behandlung der mittelschweren bis schweren, therapieresistenten Spastik bei Multipler Sklerose (MS) als Sublingualspray (Sativex) arzneimittelrechtlich zugelassen.

Cannabidiol (CBD, CBD-C₅) ist das wichtigste nicht-psychotrope Cannabinoid der Gattung Cannabis und CBD ist kein Cannabinoidrezeptoragonist.

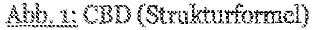

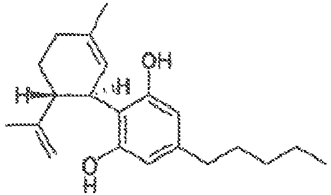

CBD kann synthetisch hergestellt werden (Michoulam R, Shvo Y., Hashish. I. The structure of cannabidiol, Tetrahedron. 1963, 19(12), 2073).

Der Auslass ist verschließbar und öffenbar. Dies bedeutet, dass der Auslass, etwa durch Anlegen eines Unterdrucks (saugen) oder durch einen mechanischen Mechanismus, geöffnet werden kann, um den gasförmigen Wirkstoff oder das gasförmige Wirkstoffgemisch zur Applikation aus dem Beutel entweichen zu lassen, und anschließend wieder geschlossen werden kann, um den verbleibenden gasförmigen Wirkstoff, bzw. das verbleibende gasförmige Wirkstoffgemisch, im Beutel zu lagern.

In einer Ausführungsform kann vorgesehen sein, dass der Auslass ein Mundstück umfasst. Erfindungsgemäß umfasst der Auslass Mittel zum Verbinden des Auslasses mit einem Mundstück. Ein Mundstück ist hierbei ein röhrenartiges Bauteil, durch das ein Gas, etwa durch Unterdruck (saugen), transportiert werden kann. Auf diese Weise wird die Applikation des freigesetzten gasförmigen Wirkstoffs erleichtert.

In diesem Zusammenhang kann vorgesehen sein, dass das Mundstück ein Teil des Auslasses ist, der Auslass samt Mundstück also ein monolithisches Bauteil ist, dass mit dem Beutel verbunden ist. Ebenso kann vorgesehen sein, dass der Auslass und das Mundstück zwei verschiedene Teile sind, die miteinander verbunden werden können, etwa durch Anschrauben, Anklicken etc. des Mundstücks an den Auslass. Darüber hinaus kann vorgesehen sein, dass die Kombination aus Auslass und Mundstück weitere Bauteile umfasst, etwa drehbare Manschetten, Steckverbindungen, Scharniere und weitere Bauteile, die dem Verbinden dienen. Insbesondere kann vorgesehen sein, dass der Auslass, sofern dieser das Mundstück nicht monolithisch umfasst, ferner eine Mundstückhalterung umfasst. Es kann vorgesehen sein, dass das Mundstück und/oder der Auslass mehrteilig sind, wobei die einzelnen Teile, die das Mundstück und/oder den Auslass bilden, miteinander verbindbar sind.

In einer weiteren Ausfuhrungsform kann vorgesehen sein, dass das Mundstück einen Lufteinlass umfasst. Ferner kann vorgesehen sein, dass das Mundstück zusätzlich eine Lufteinlassregulierung umfasst. Auf diese Weise kann die Konzentration der Menge an Umgebungsluft und gasförmigem Wirkstoff aus dem Beutel in einfacher Weise reguliert werden.

In einer Ausdrucksform kann vorgesehen sein, dass der Auslass eine Durchstechkanüle und/oder eine Durchstechmembran umfasst. Hierdurch kann besonders effektiv gewährleistet werden, dass kein Wirkstoff (vor der Applikation des gasförmigen Wirkstoffs oder des gasförmigen Wirkstoffgemischs) ungewollt entweicht.

Die Mittel zum Freisetzen eines gasförmigen Wirkstoffs oder eines gasförmigen Wirkstoffgemischs aus dem Wirkstoffvorläufer können außerhalb des Beutels oder innerhalb des Beutels angeordnet sein. Erfindungsgemäß stellen die Mittel zum Freisetzen eines gasförmigen Wirkstoffs oder eines gasförmigen Wirkstoffgemischs aus dem Wirkstoffvorläufer einen Teil des Beutels dar. In einer Ausführungsfonn kann vorgesehen sein, dass die Mittel zum Freisetzen eines gasförmigen Wirkstoffs oder eines gasförmigen Wirkstoffgemischs in dem Beutel angeordnet sind und/oder mit dem Beutel verbunden sind.

Prinzipiell sind die Mittel zum Freisetzen eines gasförmigen Wirkstoffs oder eines gasförmigen Wirkstoffgemischs aus dem Wirkstoffvorläufer nicht beschränkt, solange sie es erlauben, aus dem festen und/oder flüssigen Wirkstoffvorläufer einen relevanten pharmakologisch aktiven Wirkstoff freizusetzen. Geeignet sind die folgenden Mittel, bzw. Mittel, die es erlauben, einen oder mehrere der folgenden Verfahrensschritte durchzuführen:
- Extraktion
- Destillation
- Pressung
- Fraktionierung
- Reinigung
- Anreicherung
- Fermentation
- Temperatur
- Ultraschall
- Elektromagnetische Wellen
- Bakterien
- Pilze

Das Heizelement kann etwa eine Heizspirale, vorzugsweise aus Platin oder Goldfolie, umfassen. Die Metallfolie hat eine ähnliche Funktion wie ein Heizdraht in einer Glühbirne, kann aber nicht durchbrennen. Eine in dem Beutel platzierte Heizspirale kann über einen Akku, meist mit einer Lithium-Ionen-Zelle, mit Energie versorgt werden.

Ein chemisches Heizelement ist ein Heizelement, in dem Wärme durch eine exotherme Reaktion einer oder mehrerer in dem Heizelement enthaltener Verbindungen (wärmeerzeugende Zusammensetzung; wärmeerzeugendes Reaktionsmittel) erzeugt wird. Beispielsweise kann vorgesehen sein, dass als Reaktionsmittel ein Säureanhydrid oder ein saures Salz und ein basisches Anhydrid oder ein basisches Salz verwendet wird, wobei das basische Salz aus der Gruppe ausgewählt sein kann, welche aus Natriumacetat, Natriumbenzoat und Kaliumascorbat besteht. Ferner umfasst sein kann ein inertes Material, das aus der Gruppe gewählt ist, die aus einem Öl, einem Wachs, einem oberflächenaktiven Mittel besteht.

In dem Fall, dass in dem chemischen Heizelement zwei verschiedene Verbindungen enthalten sind, die miteinander in einer exothermen Reaktion reagieren, kann vorgesehen sein, dass, vor der Aktivierung, diese Verbindungen in unterschiedlichen Reservoirs vorliegen, die ein ungewolltes Vermischten und Reagieren der Verbindungen verhindern. Durch Betätigung eines Öffnungselements werden die Reservoirs geöffnet, so dass die beiden Reaktionsmittel in Kontakt kommen und reagieren und Wärme frei wird. Eine Berührung beider Materialien führt zu einer exothermen chemischen Reaktion. Alternativ kann ein erstes Reservoir auch einen Brennstoff, wie beispielsweise Alkohol, und die das zweite Reservoir ein Oxidationsmittel, wie etwa Permanganatverbindungen, enthalten, so dass bei Berührung beider Materialien ebenfalls eine wärmeerzeugende chemische Reaktion abläuft. Die Verwendung eines chemischen Heizelements erlaubt eine von einer Stromzufuhr unabhängige Verwendung des erfindungsgemäßen Kits.

In einer Ausführungsform kann vorgesehen sein, dass der Beutel eine Folie umfasst, vorzugsweise gefertigt aus einem oder mehreren Kunststoffen, vorzugsweise ausgewählt aus der Gruppe bestehend aus Polyethylenterephtalat, Polyethylenterephtalat-Copolyester mit Isophthalsäure und Diethylenglycol, Polyethylennaphthalat, Polyethylenfuranoat, Polylactid und Mischungen davon. Hierdurch kann eine sichere und einfache Beutelfabrikation erreicht werden.

In einer weiteren Ausführungsform kann vorgesehen sein, dass der Beutel eine erste Kammer und eine zweite Kammer umfasst, wobei die ersten Kammer den festen und/oder flüssigen Wirkstoffvorläufer beinhaltet und mit der zweiten Kammer verbunden ist; und der Auslass an der zweiten Kammer angeordnet ist. Hierdurch kann verhindert werden, dass, in unerwünschter Weise feste und/oder flüssige Teile des Wirkstoffvorläufers zum Auslass gelangen.

Die erste Kammer kann hierbei aus einer thermoplastischen Folie, insbesondere PET, PET Copolyester mit Isophthalsäure und Diethylenglykol, PET, PEN, PEF, PLA hergestellt sein. Die zweite Kammer kann aus einer ein-, zwei-oder noch mehr Schichten aufweisenden Folie- oder Aluminiumfolie, einer Aluminiumfolie, gegebenenfalls ein- oder zweiseitig Folie beschichtet, oder Ähnlichem, gefertigt sein.

In einer weiteren Ausführungsform kann vorgesehen sein, dass der Beutel ferner einen Anschluss umfasst, geeignet, um einen Zusatztank mit dem Beutel zu verbinden. In dem Zusatztank kann etwa Sauerstoff oder andere gasförmige Substanzen gelagert werden. Der Anschluss ermöglicht es, diese zusätzlichen Substanzen in einfacher Weise in den Beutel einzubringen.

In einer weiteren Ausführungsform kann vorgesehen sein, dass der Beutel eine Durchlässigkeit für Kohlendioxid von mehr als 1.0 ^{∗}10⁻² ml/(cm² *atm * Tag) und bevorzugt mehr als 2.0 ^{∗}10⁻² m)/(cm^{2 ∗}atm * Tag) besitzt. Ebenso kann vorgesehen sein, dass der Beutel eine Gasdurchlässigkeit für den gasförmigen Wirkstoff, bzw. das gasförmige Wirkstoffgemisch von weniger als 10 ^{∗}10⁻² ml/(cm² *atm * Tag) hat.

Darüber hinaus können eine Vielzahl von weiteren Ausführungsformen zur vorteilhaften Gestaltung der Erfindung beitragen.

Es kann vorgesehen sein, dass der Beutel, insbesondere der Auslass desselben, eine Kindersicherung umfasst.

Es kann vorgesehen sein, dass der Beutel aus einem Gewebe, insbesondere unter Verwendung von Carbonfasern, gefertigt ist.

Es kann vorgesehen sein, dass der Beutel aus einer Folie besteht.

Es kann vorgesehen sein, dass der Beutel aus einem Material gefertigt ist, das mit einer Farbveränderung auf eine Änderung der Temperatur reagiert.

Es kann vorgesehen sein, dass das Kit ferner ein Thermometer umfasst, das die Temperatur innerhalb des Beutels anzeigt.

Es kann vorgesehen sein, dass das Heizelement durch Quecksilberdampf gefertigt wird.

Es kann vorgesehen sein, dass Wärme in dem Heizelement, insbesondere in dem chemischen Heizelement, durch einen Katalysatoreffekt erzeugt wird.

Es kann vorgesehen sein, dass der feste Wirkstoffvorläufer gemahlene Cannabisblüten, vorzugsweise in einer mehlförmigen Ausgestaltung, umfasst. Erfindungsgemäß ist der feste und/oder flüssige Wirkstoffvorläufer zumindest teilweise in Form einer Beschichtung auf der Innenseite des Beutels angeordnet. Insbesondere kann vorgesehen sein, dass die Innenseite des Beutels mit gemahlenen Cannabisblüten, insbesondere in einer mehlförmigen Ausgestaltung, beschichtet ist. Hierdurch wird eine besonders hohe spezifische Oberfläche des Wirkstoffvorläufers und damit eine erleichterte Gasfreisetzung erreicht.

Es kann vorgesehen sein, dass der gasförmige Wirkstoff, bzw. das gasförmige Wirkstoffgemisch, ein Medikament und/oder Impfstoff ist. Hierdurch kann beispielsweise ein natürliches Verfahren erreicht werden.

Es kann vorgesehen sein, dass das Kit eine Treibmittelzusammensetzung in Gestalt einer Heizeinheit, Nährlösung und Mikroorganismen, oder Kräuter u. Blüten, umfasst.

In einer weiteren Ausführungsform kann vorgesehen sein, dass die erste Kammer aus einer barrierefreien thermoplastischen Folie hergestellt ist, und in dieser Kräuter , Blüten und Pflanzenbestandteile oder Nährlösung und Mikroorganismen aufgenommen sind, wobei die Mikroorganismen in der Lage sind, durch Gärung Wirkstoffe freizusetzen, und der zweite Behälter am Auslass angeordnet ist und der Aufnahme und Mischung der auszugebenden Zusammensetzung dient und/oder eine Mehrzahl von zweiten Behältern ineinander vorgesehen sind, welche beispielsweise die Gestalt von Kugeln besitzen können.

Es kann vorgesehen sein, dass der Beutel gasdicht ist.

Es kann vorgesehen sein, dass in den Beutel der feste und/oder flüssige Wirkstoffvorläufer zusammen mit Funktionselementen (wobei die Funktionselemente die Mittel zum Freisetzen eines gasförmigen Wirkstoffs oder eines gasförmigen Wirkstoffgemischs umfassen können, jedoch nicht notwendigerweise auf diese beschränkt sind) eingelegt wird und die Funktionselemente von außen angesteuert werden können, um einer Freisetzung des gasförmigen Wirkstoffs bzw. der Freisetzung des gasförmigen Wirkstoffgemischs zu dienen.

Es kann vorgesehen sein, dass die Innenseite des Beutels mit einer Beschichtung versehen ist, die geeignet ist, gasförmige Substanzen, die nicht der gasförmige Wirkstoff sind, bzw. nicht in dem gasförmigen Wirkstoffgemischs enthalten sind, zu binden.

Die Aufgabe wird ferner gelöst durch ein Verfahren zum Erzeugen eines gasförmigen Wirkstoffs oder eines gasförmigen Wirkstoffgemischs, umfassend die Schritte: a) Bereitstellen eines Kits nach einem der vorangehenden Ansprüche; b) Bereitstellen des festen und/oder flüssigen Wirkstoffvorläufer in dem Beutel; und c) Freisetzen des gasförmigen Wirkstoffs oder des gasförmigen Wirkstoffgemischs aus dem festen und/oder flüssigen Wirkstoffvorläufer unter Zuhilfenahme der Mittel zum Freisetzen eines gasförmigen Wirkstoffs oder eines gasförmigen Wirkstoffgemischs.

Hierbei kann vorgesehen sein, dass das Freisetzen das Erhitzen des festen und/oder flüssigen Wirkstoffvorläufers umfasst.

In diesem Zusammenhang kann vorgesehen sein, dass der feste und/oder flüssige Wirkstoffvorläufer während des Erhitzen auf eine Temperatur in einem Bereich von 170 bis 220 °C, vorzugsweise 180°C bis 210°C, besonders bevorzugt 185 bis 210 °C, erhitzt wird.

Wer Cannabis zu medizinischen Zwecken zu sich nimmt, will möglichst viele Cannabinoide (vor allem THC und CBD) aus der Hanfpflanze lösen. Diese Cannabinoide liegen in der Hanfpflanze jedoch nicht in ihrer wirksamen Reinform, sondern als sogenannte Carboxylsäuren (THCA und CBDA) vor. Diese Carboxylsäuren müssen zu THC und CBD umgewandelt werden. Dies wird mittels der sogenannten Decarboxylierung erreicht. Bei diesem Prozess wird von den Carboxylsäuren THCA und CBDA jeweils ein Molekül Kohlendioxid abgespaltet. Übrig bleiben die gewünschten Verbindungen THC und CBD.

Die Decarboxylierung kann mit Hitze eingeleitet werden. Deshalb ist beim Verdampfen von Cannabis mittels der vorliegenden Erfindung die Einstellung der richtigen Verdampfungstemperatur durch elektronische Einstellung gewährleistet. Diese sollte zwischen bevorzugt zwischen 180 und 210 Grad Celsius liegen.

Folgende Verfahrenschritte können angewendet werden. Cannabis wird zunächst mit elektromagnetischen Wellen auf genau 180 °Celsius erhitzt, Sensoren steuern die Temperatur auf 1° genau. Die Temperatur kann auf 210 °C gesteigert werden, um möglichst alle Cannabinoide zu lösen. Natürlich ist auch die Verwendung von Cannabisöl oder Extrakten möglich.

Ein weiterer Vorteil des erfindungsgemäßen Verfahrens: Die Wohnung bleibt geruchfrei. Wer in seiner Wohnung Cannabis raucht, muss damit rechnen, dass der Geruch des Rauches noch tagelang wahrnehmbar sein kann. Beim Verdampfen besteht diese Gefahr nicht.

Das Verbrennen der Blüten kann dadurch verhindert werden, dass der Erhitzungsprozess unter Ausschluss von Sauerstoff stattfindet.

Cannabisblüten können vor der Verwendung zerkleinert werden. Hierfür eignen Mahlkugeln die mit Hilfe von Magneten bewegt werden. können. Eine Steigerung des Effektes wird dadurch erreicht, dass die Metallkugeln induktiv beheizt werden.

Erfindungsgemäß kann je nach Therapie Cannabis mit anderen Heilpflanzen kombiniert werden. Das ist mit dem vorliegenden Verfahren leicht zu realisieren, weil der Beutel mehrfach mit verschiedenen Kammern kombiniert werden kann. Durch den Entourage- Effekt kann hierbei die Wirkung von Cannabis unterstützt werden.

Es kann vorgesehen sein, dass das Kit weitere Funktionselemente umfasst. Beispielhaft seien hier die folgenden Funktionselementen genannt
- Material zum Binden von Flüssigkeit
- Filtermaterialien
- Heizelemente
- Mechanische Mahlwerke
- alle Materialien die eine Chemische oder mechanische Reaktion ausüben
- Elemente die sich statisch aufladen
- Zentrifuge
- Katalysator
- UV Filter

Schließlich wird er die Aufgabe gelöst durch eine gasförmige Zusammensetzung erhältlich durch das erfindungsgemäße Verfahren, wobei der Wirkstoffvorläufer eine Cannabisblüte, Marihuana, Haschisch, Haschischöl, zumindest ein Cannabinoid oder eine Mischung davon umfasst; und der feste und/oder flüssige Wirkstoffvorläufer während des Erhitzen auf eine Temperatur in einem Bereich von 170 bis 220 °C, vorzugsweise 180°C bis 210°C, besonders bevorzugt 185 bis 210 °C, erhitzt wird.

### DETAILLIERTE BESCHREIBUNG DER ERFINDUNG

Im Folgenden soll die Erfindung unter Bezugnahme auf die Zeichnungen anhand konkreter Ausführungsformen beschrieben werden. Hierbei ist zu verstehen, dass die Bezugnahme auf die konkreten Ausführungsformen lediglich der Veranschaulichung der Erfindung dient. Die Merkmale der konkreten Ausführungsformen sind nicht notwendigerweise limitierend für die Erfindung, können jedoch, insbesondere in Kombination mit den im Vorangehenden genannten Ausfuhrungsformen, zur vorteilhaften Verwirklichung der Erfindung beitragen.

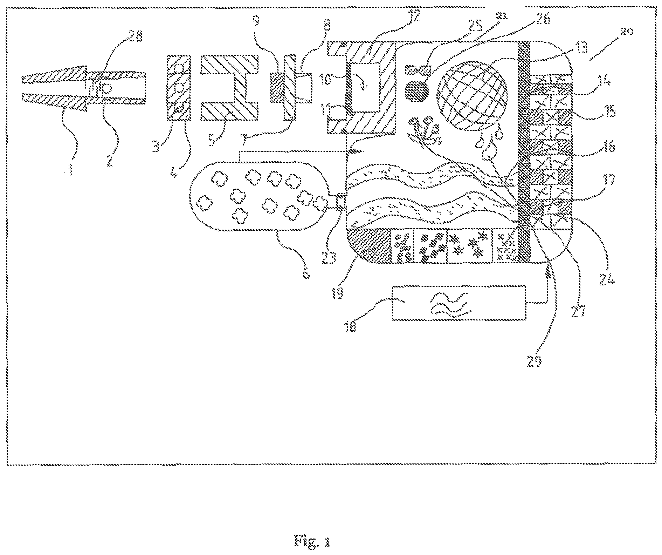

Fig.1: schematische Darstellung eines Kits gemäß einer Ausfiihrungsform der Erfindung.

In der Figur 1 kommt den Bezugszeichen die folgende Bedeutung zu:
1 Mundstück
2 Lufteinlass
3 Lufteinlassregulierung
4 Manschette, drehbar
5 Mundstückhalterung
6 Zusatztank
7 Steckverbindung
8 Durchstechkanüle
9 Verbindungselement
10 Durchstechmembran
11 Scharnier
12 Auslass
13 feinmaschiges Körbchen für Blüten oder anderes Biomaterial
14 Chemisches Heizelement
15 Heizelemente
16 Flüssige Wirkstoffe
17 in den Beutel eingearbeitete Wirkstoffe
18 Externer Energieerzeuger
19 Knickelement, löst chemische Reaktion aus
20 Kit
21 Beutelhülle
23 Anschluss für externes Befüllen
24 Heizelement
25 Metallblättchen zum Knicken, löst chemische Reaktion aus
26 Schadstoffsauger mit Aktivkohlefilter
27 Biomaterial, etwa Cannabis Blüte
28 Filter
29 flüssige Wirkstoffe

In der in Figur 1 gezeigten Ausführungsform werden eine Vielzahl von Merkmalen und Elementen, die zur vorteilhaften Realisierung der Erfindung beitragen können, gleichzeitig gezeigt. Der Fachmann wird verstehen, dass nicht notwendigerweise sämtliche der in der Figur 1 gezeigten Merkmale (Bauteile etc.) zur Realisierung einer erfindungsgemäßen Vorrichtung gleichzeitig vorhanden sein müssen.

In der Figur 1 wird ein erfindungsgemäßes Kit 20 gezeigt. Dieses umfasst. einen Beutel 21 mit einem Auslass 12 und verschiedene Mitteln 14, 15, 18, 19, 24, 25 zum Freisetzen eines gasförmigen Wirkstoffs oder eines gasförmigen Wirkstoffgemischs aus dem Wirkstoffvorläufer. In dem Beutel 21 sind feste Wirkstoffvorläufer 27 und flüssige Wirkstoffvorläufer 16, 29 enthalten. Handelt es sich bei dem festen Wirkstoffvorläufer 27 um ein Material, das fein verteilt vorliegt, etwa ein pulverförmiges Material, kann dieses in einem feinmaschigen Körbchen 13 vorliegen. Weiterer fester Wirkstoffvorläufer ist in einen Teil des Beutels 17 eingearbeitet Durch mechanische Einwirkung auf den Teil des Beutels 17 kann der darin enthaltene Wirkstoffvorläufer freigesetzt werden.

Der in der Figur 1 gezeigt Auslass 12 umfasst eine Durchstechmembran 10 und ein Scharnier 11, dass die Durchstechmembran 10 mit dem Auslass 12 in einer Art und Weise verbindet, dass diese beweglich angeordnet ist. Der Auslass 12 ist gestaltet, um mit einem Mundstück 1 und weiteren Teilen, nämlich einer Manschette 4, einer Mundstückhalterung 5 und einer Steckverbindung 7, verbunden werden zu können. Das Mundstück 1 umfasst einen Lufteinlass 2 sowie einen Filter 28. Mit der Manschette 4 wird eine Lufteinlassregulierung 3 zwischen dem Mundstück 1 und dem Auslass 12 angeordnet. Zusammen mit der Steckverbindung 7 wird ein Verbindungselement 9 und eine Durchstechkanüle 8 zwischen dem Auslass 12 und der Mundstückhalterung 5 angeordnet.

In der Figur 1 werden verschiedene Mittel zum Freisetzen eines gasförmigen Wirkstoffs oder eines gasförmigen Wirkstoffgemischs aus dem Wirkstoffvorläufer gezeigt. Gezeigt wird etwa ein externer Energieerzeuger 18, der eine außerhalb des Beutels angeordnete Heizung sein kann. Ferner gezeigt werden chemische Heizelemente 14, weitere Heizelemente 15, ein Knickelement 19, dass eine exotherme chemische Reaktion auslösen kann, ein weiteres Heizelement 24 sowie eine Metallplättchen 25, dass geknickt werden kann, um eine exotherme chemische Reaktion auszulösen.

Darüber hinaus umfasst der Beutel 21 einen Schadstoffsauger 26, der mit einem Aktivkohlefilter versehen ist. Schließlich weist der Beutel einen Anschluss 23 auf, der ein Befüllen des Beutels 21 mit Material aus einem Zusatztank 6 erlaubt.

Die in der vorangehenden Beschreibung und den beigefügten Ansprüchen offenbarten Eigenschaften können, separat oder in einer Kombination, Gegenstand zur Realisierung der Aspekte der in den unabhängigen Ansprüchen gemachten Offenbarung in unterschiedlichen Formen davon sein.

## Patentansprüche

1. Kit, um einen pharmakologisch aktiven Wirkstoff in der Lunge aufzunehmen, umfassend:
a. einen Beutel (21) mit einem Auslass (12), wobei der Auslass (12) verschließbar und öffenbar ist, und der Auslass Mittel zum Verbinden des Auslasses mit einem Mundstück umfasst;
b. einen festen und/oder flüssigen Wirkstoffvorläufer (16,27,29); und
c. Mittel (14, 15, 18, 19, 24, 25) zum Freisetzen eines gasförmigen Wirkstoffs oder eines gasförmigen Wirkstoffgemischs aus dem Wirkstoffvorläufer, wobei
d. der feste und/oder flüssige Wirkstoffvorläufer (16, 27, 29) zumindest teilweise in Form einer Beschichtung auf der Innenseite des Beutels (21) angeordnet ist, und
e. die Mittel (14, 15, 18, 19, 24, 25) zum Freisetzen eines gasförmigen Wirkstoffs oder eines gasförmigen Wirkstoffgemischs ein Heizelement (14, 15, 24), vorzugsweise ein chemisches Heizelement (14), umfassen, **dadurch gekennzeichnet, dass** die Mittel zum Freisetzen eines gasförmigen Wirkstoffs oder eines gasförmigen Wirkstoffgemischs aus dem Wirkstoffvorläufer einen Teil des Beutels darstellen.

2. Kit nach Anspruch 1, wobei der Wirkstoffvorläufer eine Cannabisblüte, Marihuana, Haschisch, Haschischöl, zumindest ein Cannabinoid oder eine Mischung davon umfasst.

3. Kit nach einem der vorangehenden Ansprüche, wobei der Auslass (12) ein Mundstück (1) umfasst.

4. Kit nach Anspruch 3, wobei der Auslass (12) Mittel (7) zum Verbinden des Auslasses (12) mit einem Mundstück (1) umfasst.

5. Kit nach einem der vorangehenden Ansprüche, wobei der Beutel (21) eine erste Kammer und eine zweite Kammer umfasst, wobei
die ersten Kammer den festen und/oder flüssigen Wirkstoffvorläufer (16, 27, 29) beinhaltet und mit der zweiten Kammer verbunden ist; und
der Auslass (12) an der zweiten Kammer angeordnet ist.

6. Verfahren zum Erzeugen eines gasförmigen Wirkstoffs oder eines gasförmigen Wirkstoffgemischs, umfassend die Schritte:
a. Bereitstellen eines Kits nach einem der vorangehenden Ansprüche;
b. Bereitstellen des festen und/oder flüssigen Wirkstoffvorläufer (16, 27, 29) in dem Beutel (1); und
c. Freisetzen des gasförmigen Wirkstoffs oder des gasförmigen Wirkstoffgemischs aus dem festen und/oder flüssigen Wirkstoffvorläufer (16, 27, 29) unter Zuhilfenahme der Mittel zum Freisetzen eines gasförmigen Wirkstoffs oder eines gasförmigen Wirkstoffgemischs.

7. Verfahren nach Anspruch 6, wobei das Freisetzen das Erhitzen des festen und/oder flüssigen Wirkstoffvorläufers (16, 27, 29) umfasst.

8. Verfahren nach Anspruch 7, wobei der feste und/oder flüssige Wirkstoffvorläufer (16, 27, 29) während des Erhitzen auf eine Temperatur in einem Bereich von 170 bis 220 °C, vorzugsweise 185 bis 210 °C, erhitzt wird.

## Claims

1. A kit to incorporate a pharmacologically active agent in the lungs, comprising:
a. a bag (21) having an outlet (12), the outlet (12) being closable and openable, the outlet comprising means for connecting the outlet to a mouthpiece;
b. a solid and/or liquid active substance precursor (16, 27, 29); and
c. means (14, 15, 18, 19, 24, 25) for releasing a gaseous active substance or a gaseous mixture of active substances from the active substance precursor,
wherein
d. the solid and/or liquid active substance precursor (16, 27, 29) is arranged at least partially in the form of a coating on the inside of the bag (21), and
e. the means (14, 15, 18, 19, 24, 25) for releasing a gaseous active substance or a gaseous active substance mixture comprise a heating element (14, 15, 24), preferably a chemical heating element (14), **characterized in that** the means for releasing a gaseous active substance or a gaseous active substance mixture from the active substance precursor constitute part of the bag.

2. The kit of claim 1, wherein the active substance precursor comprises a cannabis flower, marijuana, hashish, hashish oil, at least one cannabinoid, or a mixture thereof.

3. The kit according to any one of the preceding claims, wherein the outlet (12) comprises a mouthpiece (1).

4. The kit of claim 3, wherein the outlet (12) comprises means (7) for connecting the outlet (12) to a mouthpiece (1).

5. The kit according to any one of the preceding claims, wherein the bag (21) comprises a first chamber and a second chamber, wherein
said first chamber contains said solid and/or liquid substance precursor (16, 27, 29) and is connected to said second chamber, and
the outlet (12) is arranged at the second chamber.

6. A method for producing a gaseous active substance or a gaseous active substance mixture, comprising the steps:
a. Providing a kit according to any one of the preceding claims;
b. Providing the solid and/or liquid active substance precursor (16, 27, 29) in the bag (1); and
c. Releasing the gaseous active substance or the gaseous active substance mixture from the solid and/or liquid active substance precursor (16, 27, 29) with the aid of the means for releasing a gaseous active substance or a gaseous active substance mixture.

7. The method of claim 6, wherein said releasing comprises heating the solid and/or liquid active substance precursor (16, 27, 29).

8. The method of claim 7, wherein the solid and/or liquid active substance precursor (16, 27, 29) is heated to a temperature in a range of 170 to 220 °C, preferably 185 to 210 °C, during the heating.

## Revendications

1. Kit contenant une substance pharmacologiquement active dans le poumon, comprenant :
a. un sachet (21) avec une sortie (12), dans lequel la sortie (12) peut être fermée et ouverte et la sortie comprend des moyens pour le raccordement de la sortie avec une embouchure ;
b. un précurseur de substance active solide et/ou liquide (16, 27, 29) ; et
c. des moyens (14, 15, 18, 19, 24, 25) pour la libération d'une substance active gazeuse ou d'un mélange de substances actives gazeuses à partir du précurseur de substance active,
dans lequel
d. le précurseur de substance active solide et/ou liquide (16, 27, 29) est disposé sur la face interne du sachet (21) au moins partiellement sous la forme d'un revêtement et
e. les moyens (14, 15, 18, 19, 24, 25) pour la libération d'une substance active gazeuse ou d'un mélange de substances actives gazeuses comprennent un élément chauffant (14, 15, 24), de préférence un élément chauffant chimique (14),
**caractérisé en ce que** les moyens pour la libération d'une substance active gazeuse ou d'un mélange de substances actives gazeuses à partir du précurseur de substance active constituent une partie du sachet

2. Kit selon la revendication 1, dans lequel le précurseur de substance active comprend une fleur de cannabis, de la marijuana, du haschisch, de l'huile de haschisch,au moins un cannabinoïde ou un mélange de ceux-ci.

3. Kit selon l'une des revendications précédentes, dans lequel la sortie (12) comprend une embouchure (1).

4. Kit selon la revendication 3, dans lequel la sortie (12) comprend des moyens (7) pour le raccordement de la sortie (12) avec une embouchure (1).

5. Kit selon l'une des revendications précédentes, dans lequel le sachet (21) comprend une première chambre et une deuxième chambre, dans lequel
la première chambre contient le précurseur de substance active solide et/ou liquide (16, 27, 29) et est reliée avec la deuxième chambre ; et
la sortie (12) est disposée au niveau de la deuxième chambre.

6. Procédé de production d'une substance active gazeuse ou d'un mélange de substances actives gazeuses, comprenant les étapes suivantes :
a. fourniture d'un kit selon l'une des revendications précédentes ;
b. fourniture d'un précurseur de substance active solide et/ou liquide (16, 27, 29) dans le sachet (1) ; et
c. libération de la substance active gazeuse ou du mélange de substances actives gazeuses à partir du précurseur de substance active solide et/ou liquide (16, 27, 29) à l'aide des moyens pour la libération d'une substance active gazeuse ou d'un mélange de substances actives gazeuses.

7. Procédé selon la revendication 6, dans lequel la libération comprend le chauffage du précurseur de substance active solide et/ou liquide (16, 27, 29).

8. Procédé selon la revendication 7, dans lequel le précurseur de substance active solide et/ou liquide (16, 27, 29) est chauffé, pendant le chauffage, à une température de l'ordre de 170 à 200 °C, de préférence de 185 à 210 °C.
